# EUROPEAN PATENT SPECIFICATION

(11) **EP 0 441 116 B1**
(45) Date of publication and mention of the grant of the patent: **05.04.1995**
(21) Application number: 91100082.6
(22) Date of filing: 02.01.1991
(51) Int. Cl.: C07D 311/72, C07C 43/23, C07D 493/08, C07D 309/10, C07C 50/28, C07C 46/02

(54) **Process and intermediates for the manufacture of optically active chroman derivatives**
Verfahren und Zwischenprodukte zur Herstellung von optisch aktiven Chromanderivaten
Procédé et intermédiaires pour la fabrication des dérivés de chromanes optiquement actifs

(30) Priority: 18.01.1990 JP 9157/90
(43) Date of publication of application: 14.08.1991
(73) Proprietor: ASAHI DENKA KOGYO KABUSHIKI KAISHA, Arakawa-ku Tokyo 116 (JP)
(72) Inventor: Takano, Seiichi, Sendai-shi, Miyagi-ken, 981-31 (JP); Ogasawara, Kunio, Sendai-shi, Miyagi-ken, 981 (JP)
(74) Representative: VOSSIUS & PARTNER

(56) References cited:
- EP-A- 0 122 426
- TETRAHEDRON LETTERS, vol. 31, no. 25 1990, pages 3619-3622, Oxford, GB; S.TAKANO et al.: "A convergent enantiocontrolled route to mevalonolactone andvitamin E from (S)-O-benzylglycidol"

## Description

The present invention relates to an optically active novel compound and intermediate products thereof, and a process for the manufacture of same.

Vitamin E is a methylated derivative of a tocopherol, and includes eight kinds of naturally occurring compounds, i.e., α-, β-, γ-, and δ-tocopherols, and α-, β-, γ-, and δ-tocotrienols. Although tocopherols and tocotrienols include d-form, l-form or dl-form optical isomers, naturally occurring compounds have optical activities. Synthesized tocopherols are generally prepared in the form of a diastereomer, and it is known that the chirality of the carbon atom of the 2-position in the chroman ring has a considerable effect on the physiological activities of tocopherols.

A method of synthesizing optically active tocopherols from optically active starting materials is described in, for example, N. Cohen, et al, Journal of the American Chemical Society, 101:22, October 24, 1979, 6710-6716. In the method of Cohen, et al, an optically active desired α-tocopherol is obtained by using an optically active benzopyran derivative as a starting material, to form an optically active chroman-2-methanol derivative, and then performing a Wittig coupling of the chroman derivative to form the final α-tocopherol while retaining the chirality of the starting material. In this method, however, a resolution process is required to obtain the optically active starting material, and poisonous hydrocyanic acid must be used during the course of the synthesis process.

Optically active chroman-2-ethanol derivatives and a process for producing them are also disclosed in EP-A-122 426. However, the process disclosed in EP-A-122 426 is not an enantio-controlled process, but for obtaining optically active chroman-2-ethanol derivatives in the (R) or (S) configuration, an additional optical resolution step is necessary.

Accordingly, an object of the present invention is to provide intermediates from which naturally occurring or non-occurring tocopherols can be alternatively prepared, without the need for a resolution process and the need of poisonous reagents, and which can be prepared from an easily available starting material having optical activity.

In accordance with the present invention, there is provided a process for manufacturing an optically active (S)- or (R)-chroman-2-ethanol compound of the general formula (I): 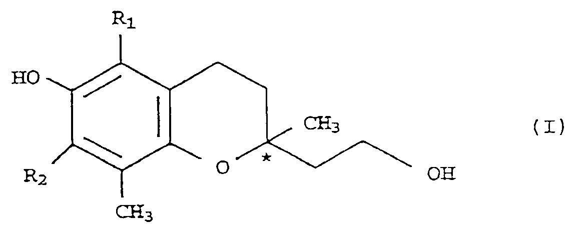
wherein R₁ and R₂ independently represent a hydrogen atom or a lower alkyl group having 1 to 4 carbon atoms, and the chiral central carbon atom marked with a symbol * in said formula (I) alternatively has one of an R-configuration and an S-configuration, said process comprising the following steps:
reducing an optically active (S)- or (R)-mevalonolactone of the formula (II): 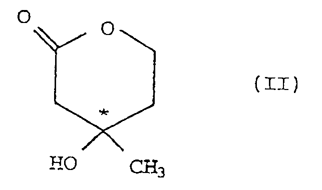
wherein the symbol * has the same meaning as above, to obtain the corresponding optically active (S)- or (R)-mevalonolactol of the formula (III): 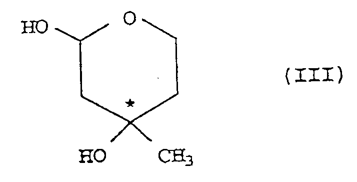
wherein the symbol * has the same meaning as above, while retaining the R-configuration or S-configuration of the chiral central carbon atom marked with the symbol * in said formula (II);
reacting the optically active (S)- or (R)-mevalonolactol of the formula (III) and a benzene magnesium halide of the general formula (IV): 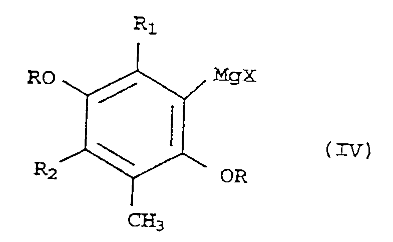
wherein R represents a lower alkyl group having 1 to 4 carbon atoms and optionally substituted with one or more lower alkoxy groups having 1 to 4 carbon atoms, a benzyl or substituted benzyl group, a substituted phenyl group, or a silyl group substituted with one or more lower alkyl groups having 1 to 4 carbon atoms, X represents a halogen atom, R₁ and R₂ have the same meanings as above, to obtain the corresponding optically active (S)- or (R)-phenylpentanetriol compound of the general formula (V): 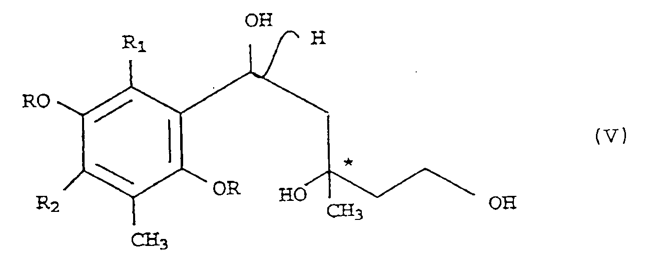
wherein R, R₁, R₂ and the symbol * have the same meanings as above, while retaining the R-configuration or S-configuration of the chiral central carbon atom marked with the symbol * in said formula (III);
removing the groups R from the optically active (S)- or (R)-phenylpentanetriol compound of the formula (V) to obtain the corresponding optically active (S)- or (R)-quinone-pentanetriol compound of the general formula (VI): 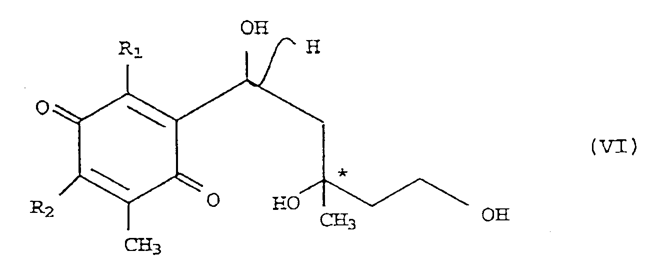
wherein R₁, R₂ and the symbol * have the same meanings as above, while retaining the R-configuration or S-configuration of the chiral central carbon atom marked with the symbol * in said formula (V);
cyclizing the optically active (S)- or (R)-quinonepentanetriol compound of the general formula (VI) to obtain the corresponding optically active tricyclic benzoquinone monoketal compound of the general formula (VII): 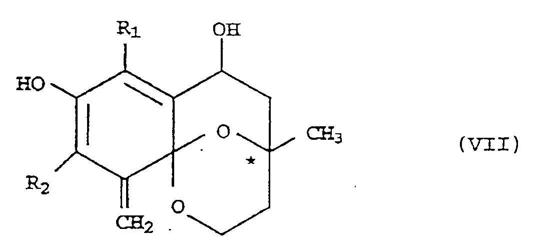
wherein R₁, R₂ and the symbol * have the same meanings as above, while retaining the R-configuration or S-configuration of the chiral central carbon atom marked with the symbol * in said formula (VI); and
treating the optically active tricyclic benzoquinone monoketal compound of the general formula (VII) under a hydrogenolysis condition, to obtain the corresponding optically active (S)- or (R)-chroman-2-ethanol compound, while retaining the R-configuration or S-configuration of the chiral central carbon atom marked with the symbol * in said formula (VII).
Further, in accordance with the present invention, there is provided an optically active (S)- or (R)-phenylpentanetriol compound of the general formula (V) 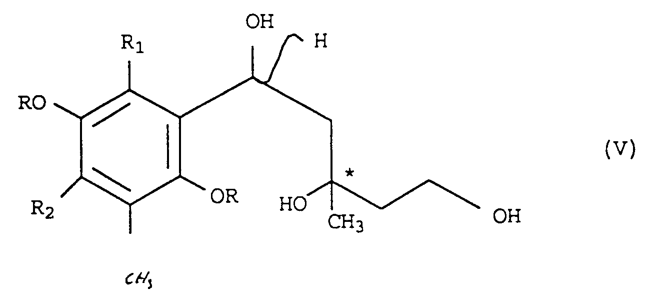
wherein R represents a group for protecting a hydroxy group, and R₁, R₂ and the symbol * have the same meanings as above.

Still further, in accordance with the present invention, there is provided an optically active (S)- or (R)-quinonepentanetriol compound of the general formula (VI) 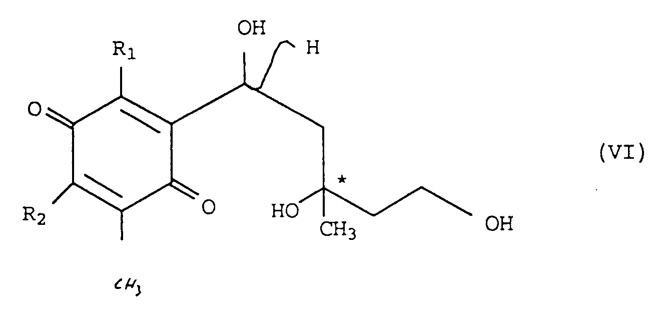
wherein R₁, R₂ and the symbol * have the same meanings as above.

Still further, in accordance with the present invention, there is also provided an optically active tricyclic benzoquinone monoketal compound of the general formula (VII) 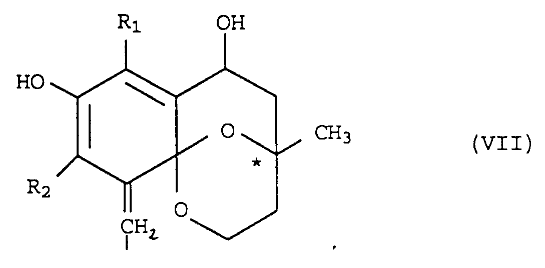
wherein R₁, R₂ and the symbol * have the same meanings as above.

Still further, in accordance with the present invention, there is provided a process for manufacturing the above compounds.

The process for manufacturing the compounds of the present invention includes the following steps.
(a) A step comprising reducing the optically active (S)- or (R)-mevalonolactone of the formula (II): 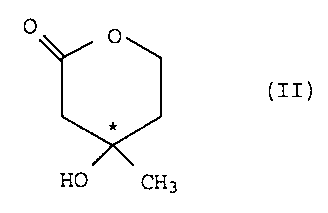 to obtain the optically active corresponding (S)- or (R)-mevalonolactol of the formula (III): 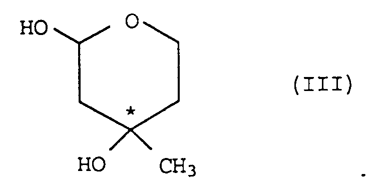
(b) A step comprising reacting the lactol of the formula (III) and a benzene magnesium halide of the formula (IV): 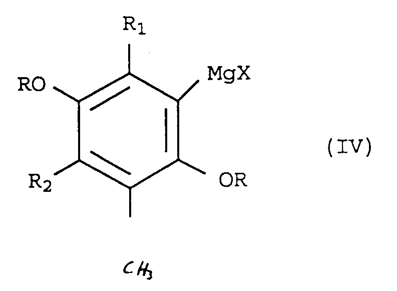 wherein R represents a group for protecting a hydroxy group, namely a lower alkyl group having 1 to 4 carbon atoms and optionally substituted with one or more lower alkoxy groups having 1 to 4 carbon atoms (such as methyl, methoxymethyl or methoxyethoxymethyl), a benzyl or substituted benzyl group, a substituted phenyl group (such as p-methoxyphenyl), or a silyl group substituted with one or more lower alkyl groups having 1 to 4 carbon atoms (such as t-butyldimethylsilyl), R₁ and R₂ independently represent a hydrogen atom or a lower alkyl group having 1 to 4 carbon atoms, preferably a methyl or ethyl group, and X represents a halogen atom, preferably a bromine or iodine atom, to thereby obtain an optically active corresponding (S)- or (R)-phenylpentanetriol compound [hereinafter optionally referred to as phenyltriol compound] of the formula (V) 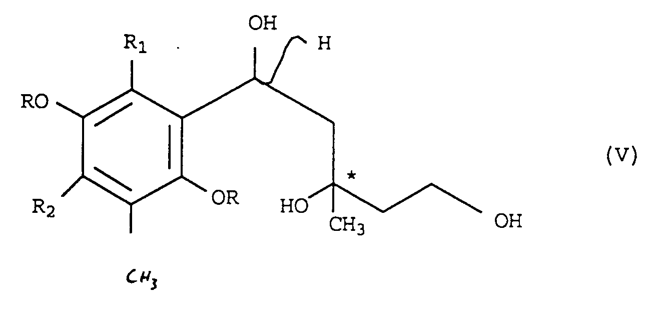 while retaining the S-configuration or R-configuration alternatively possessed by the chiral central carbon atom. In the phenyltriol compound (V) obtained in this step, the configuration of another chiral central carbon atom combined with the phenyl group is not necessarily alternative but may be in the form of an SR mixture.
(c) A step comprising removing protecting groups R from the phenyltriol compound (V), to thereby obtain an optically active corresponding (S)- or (R)-quinonepentanetriol compound [hereinafter optionally referred to as benzoquinonetriol compound] of the formula (VI): 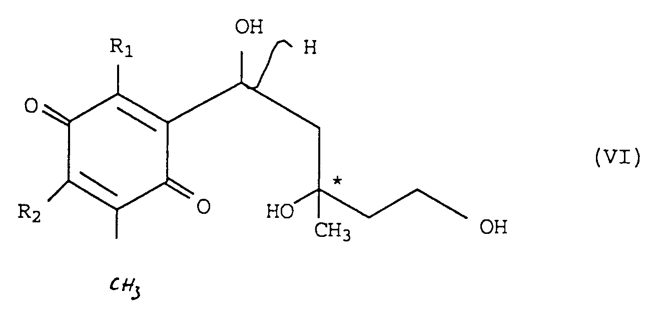 wherein R₁, R₂ and the symbol * have the same meanings as above, while retaining the S-configuration or R-configuration alternatively possessed by the chiral central carbon atom. In the benzoquinonetriol compound (VI) obtained in this step, the configuration of another chiral central carbon atom combined with the benzoquinone group is not necessarily alternative but may be in the form of an SR mixture.
(d) A step comprising cyclizing the benzoquinonetriol compound (VI), to thereby obtain an optically active corresponding tricyclic quinone monoketal compound [hereinafter optionally referred to as benzoquinone monoketal compound] of the formula (VII): 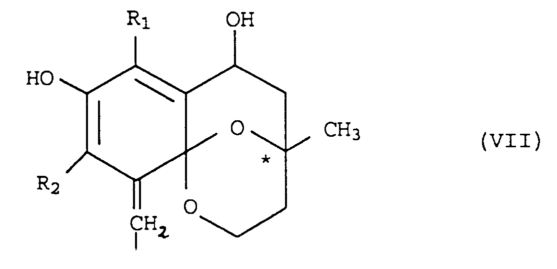 wherein R₁, R₂ and the symbol * have the same meanings as above, while retaining the S-configuration or R-configuration alternatively possessed by the chiral central carbon atom. In the benzoquinone monoketal compound (VII) obtained in this step, the configuration of another chiral central carbon atom combined with the hydroxy group in the oxane ring is not necessarily alternative but may be in the form of an SR mixture.
(e) A step comprising treating the benzoquinone monoketal compound (VII) under hydrogenolysis conditions, to thereby obtain the optically active corresponding (S)-or (R)-chroman-2-ethanol compound [hereinafter optionally referred to as chroman ethanol compound] of the formula (I), while retaining the S-configuration or R-configuration alternatively possessed by the chiral central carbon atom.

The steps (a) to (e) will be described hereinafter in detail.

### Step (a)

The starting mevalonolactone (II) contains a chiral center, and thus includes an S-form and an R-form. Each of mevalonolactones (II) in the S-form and the R-form may be prepared by the known process described in, for example, JP-A-60-146840. When the mevalonolactone (II) in the S-form or the R-form is reduced with a metal hydride reducing agent, preferably an aluminum hydride reducing agent, more preferably a dialkyl aluminum hydride (such as dibutyl aluminum hydride, or trialkoxy lithium aluminum hydride), in an atmosphere of an inert gas (such as an argon or nitrogen gas) and at a low temperature (such as 0°C to -78°C, preferably -10°C to -78°C), in an anhydrous aprotic solvent (such as tetrahydrofuran, toluene, hexane or ether), the desired mevalonolactol (III) is obtained as a waxy solid while retaining an unchanged chirality, in a substantially pure form which may be used in a subsequent step without purification.

### Step (b)

The optically active mevalonolactol (III) obtained in the above step (a) is gradually added at a low temperature of about 0°C to -20°C to the magnesium halide compound (IV) which has been previously prepared in an organic solvent (for example, ether, tetrahydrofuran or dioxane) in an atmosphere of an inert gas (such as an argon or nitrogen gas). After the reaction at about 0°C to room temperature is completed, the phenyltriol compound (V) is obtained as an oil while retaining the chirality stemming from the mevalonolactol (III). The pentane chain of the phenyltriol compound (V) includes 2 chiral central carbon atoms, i.e., (a) the chiral central carbon atom stemming from the starting mevalonolactol compound (III), and (b) a chiral central carbon atom which is newly formed by the reaction of the mevalonolactol (III) and the magnesium halide compound (IV) and which is combined with the phenyl group. The configuration of the former chiral central carbon atom (a) is maintained in the step (b). The configuration of the latter chiral central carbon atom (b) is not alternative but may be in the form of an epimer mixture of about 1:1.

The resulting compound may be used in the subsequent step, after purification (for example, by silica gel column chromatography) if necessary.

### Step (c)

The benzoquinone triol compound (VI) is obtained as an oil, while retaining an unchanged chirality, by removing the protecting group R from the phenyltriol compound (V) prepared in the above step (b), in an atmosphere of an inert gas (such as an argon or nitrogen gas), at an ordinary temperature, and in an aqueous-organic solvent preferably water-containing acetonitrile, water-containing tetrahydrofuran or water-containing dioxane, with an oxidizing agent, such as cerium (IV) salts, preferably ammonium cerium nitrate. The pentane chain of the resulting benzoquinone triol compound (VI) includes 2 chiral central carbon atoms stemming from the phenyltriol compound (V), wherein the configuration of the chiral central carbon atom (a) stemming from the starting mevalonolactol (III) is still retained in this step, and the configuration of the chiral central carbon atom (b) which is newly formed in the step (b) is still in the form of an epimer mixture of about 1:1.

The resulting compound may be used in the subsequent step, after purification (for example, by silica gel column chromatography) if necessary.

### Step (d)

The benzoquinonetriol (VI) is cyclized by an intramolecular dehydration caused by a refluxing in dioxane in the presence of a catalytic amount of sulfuric acid, to thereby obtain the tricyclic benzoquinone monoketal (VII) while retaining the chirality. In the resulting compound (VII), the configuration of the chiral central carbon atom (a) stemming from the starting mevalonolactol (III) is still retained in this step, and the configuration of the chiral central carbon atom (b), which is newly formed in the step (b) is still in the form of an epimer mixture of about 1:1. The resulting compound may be used in the subsequent step without purification, but if purification is necessary, then silica gel column chromatography is used.

### Step (e)

When the tricyclic benzoquinone monoketal (VII) is treated under hydrogenolysis conditions, the chroman-ethanol compound (I) is obtained while retaining the chirality stemming from the starting mevalonolactol (III). The resulting compound may be used in the subsequent step, after purification if necessary. The purification is carried out by silica gel column chromatography.

The chroman-ethanol compound (I) in the form of a racemic modification is known, but the optically active compounds of the S-form and R-form can not be obtained unless resolution procedure is used. The tocopherol having a naturally occurring form (i.e., R-form), which may be prepared from the chroman-ethanol compound (I) according to the present invention, exhibits a far greater physiological activity than that having the S-form.

An optically active tocopherol compound can be prepared from the optically active chroman-ethanol compound (I) according to the present invention, while retaining the chirality thereof, by effecting a benzylation of the phenolic hydroxy group in the chroman-ethanol compound (I), then a tosylation of the active hydroxy group, and thereafter, coupling of a Grignard reagent to the tosylated side chain to introduce a side chain of tocopherol, and finally, removal of the benzyl group on the phenolic hydroxy group.

According to the present invention, novel intermediate are provided, which may be prepared from an easily available mevalonolactone having an optical activity, and from which tocopherols having either a naturally occurring or non-occurring configuration can be alternatively synthesized. The intermediate compounds according to the present invention are useful as an intermediate for various antioxidizing agents. Further, the intermediate compounds of the present invention can be easily and safely prepared, because neither a resolution procedure nor poisonous reagents are required in the process for the manufacture thereof.

### Examples

The present invention now will be further illustrated by, but is by no means limited to, the following Examples.

### Example 1: Preparation of 2ξ-hydroxy-(4R)-mevalonolactol

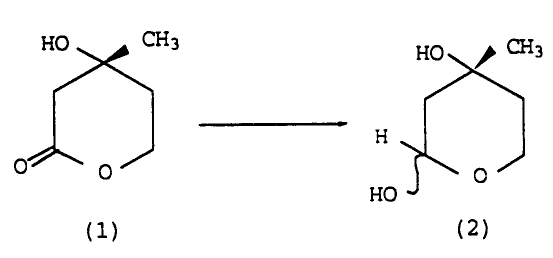

In an atmosphere of an argon stream, a toluene solution (1.5 ml: 2.7 mmol) of 1.8M diisobutylaluminum hydride was gradually added at -30°C to a tetrahydrofuran solution (4 ml) containing 211 mg (1.62 mmol) of (R)-(+)-mevalonolactone (1). The reaction mixture was stirred at -30°C for 45 minutes, quenched with 10% aqueous sodium hydroxide solution, stirred again at a room temperature for 1 hour, and then filtered with celite. Thereafter, the solvent was evaporated from the resulting filtrate to obtain an oily product. Further, the above celite used for the filtration was extracted overnight with ethyl acetate to obtain an oily product. These oily products were combined and treated with silica gel column chromatography, and from the ethyl acetate effluent, 180 mg (85 %) of the above-mentioned compound (2) was obtained as a colorless oil.
Mass analysis (m/e): 132 (M+), 68 (100%).
IR (neat) cm⁻¹: 3300.
¹H-NMR (CDCl₃) δ: 4.9 - 5.39 (1H, m), 4.4 - 3.5 (2H, m), 4.6 and 3.25 (2H, brd and brd), 2.0 - 1.4 (4H, m), 1.3, 1.25 (3H, s × 2).

### Example 2: Preparation of (3R)-1-(2,5-dimethoxy-3,4,6-triphenyl)-3-methyl-pentane-1ξ,3,5-triol

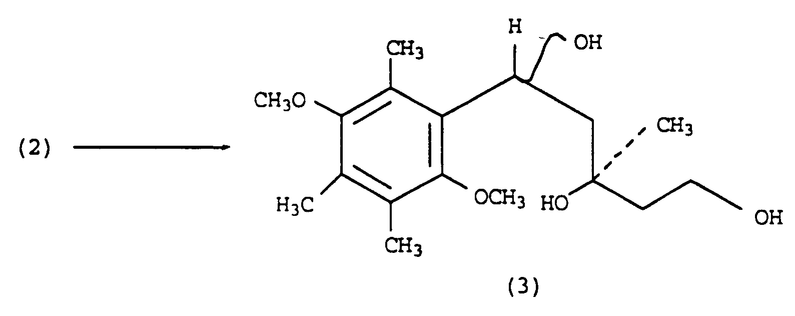

In an atmosphere of an argon stream, 1.1 g (0.045 mol) of magnesium, one drop of methyl iodide, and a catalytic amount of iodine were added to 30 ml of tetrahydrofuran, and further, a solution of 10.4 g (0.04 mol) of 2,5-dimethoxy-3,4,6-trimethyl-bromobenzene in 15 ml of tetrahydrofuran was added dropwise over 40 minutes while refluxing the tetrahydrofuran. The reaction was completed by refluxing for further 30 minutes, to thereby form 2,5-dimethoxy-3,4,6-trimethylbenzene magnesium bromide. Then, after cooling to 0°C, 580 mg (4.4 mmol) of the lactol compound (2) prepared in Example 1 was added and stirred at a room temperature for 2 hours, an aqueous sodium bicarbonate saturated solution was added to the reaction mixture, and an extraction with diethyl ether was carried out. The organic phase was washed with an aqueous sodium chloride saturated solution, and dried over anhydrous magnesium sulfate, the solvent was evaporated under a reduced pressure, and the residue was treated by silica gel column chromatography. Then, 1.35 g (98.5 %) of the above-mentioned compound (3) was obtained from the ethyl acetate effluent as a colorless oil.
Mass analysis (m/e): 312 (M+), 209 (100 %)
IR (neat) cm⁻¹: 3370
¹H-NMR (CDCl₃) δ: 5.45 (2H, m), 4.90 (2H, m), 4.25 - 3.60 (8H, m), 2.4 - 2.1 (9H, m), 1.85 - 1.5 (4H, m), 1.5 and 3.2 (3H, s × 2).

### Example 3: Preparation of (3R)-1-(3,6-dioxo-2,4,5-trimethylcyclohexadiene-1,4)-3-methyl-pentane-1ξ,3,5-triol

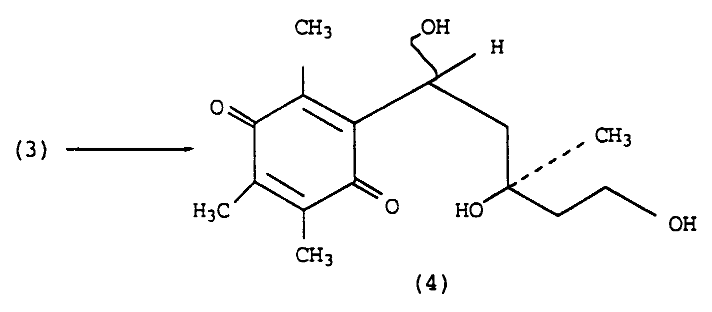

In an atmosphere of an argon stream, 1.3 g (4.17 mmol) of the triol compound (3) prepared in Example 2 was dissolved in 30 ml of acetonitrile and 30 ml of H₂O, and 11.27 g (33.4 mmol) of cerium (IV) ammonium nitrate was added at a room temperature. After stirring for 5 minutes, H₂O was added, and the reaction mixture was extracted with dichloromethane, washed successively with an aqueous sodium bicarbonate saturated solution and an aqueous sodium chloride saturated solution, and then dried over anhydrous magnesium sulfate. The solvent was evaporated under a reduced pressure, and the residue was treated by silica gel column chromatography, and 750 mg (64 %) of the above-mentioned compound (4) was obtained from the ethyl acetate/benzene (1:4) effluent as a colorless oil.
Mass analysis (m/e): 264 (-H₂O), 179 (100 %).
IR (neat) cm⁻¹: 3350, 1720, 1640.
¹H-NMR (CDCl₃) δ: 5 (1H, m), 4.7 -3.7 (5H, m), 2.4 - 2.0 (11H, m), 1.9 - 1.6 (2H, m), 1.5 and 1.3 (3H, s × 2).

### Example 4: Preparation of 6,8-dihydroxy-10-methylene-4,7,9-trimethyl-2,3,4,5,6,10,10a-heptahydro-1-benzocine

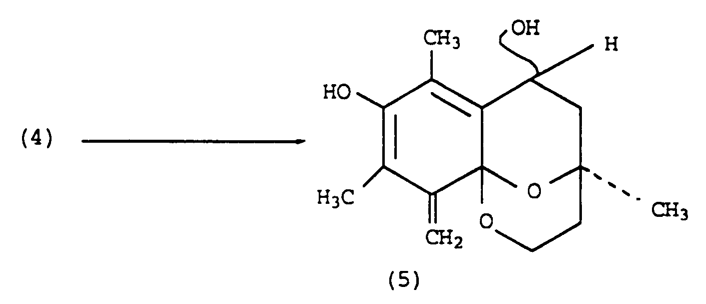

In an atmosphere of an argon stream, 345 mg (1.22 mmol) of the benzoquinone compound (4) prepared in (Example 3 was dissolved in 12 ml of dioxane, and 2 ml of 2N sulfuric acid was added. After refluxing for 3 hours, the reaction mixture was cooled to a room temperature, diethyl ether and an aqueous sodium bicarbonate saturated solution were added, and the reaction mixture was extracted with diethyl ether, washed with an aqueous sodium chloride saturated solution, and then dried over anhydrous magnesium sulfate. The solvent was evaporated under a reduced pressure to obtain the above-mentioned compound (5), which was used in the next step without purification.
Mass analysis (m/e): 264 (M+), 201 (100 %).
IR (neat) cm⁻¹: 3350.
¹H-NMR (CDCl₃) δ: 7.0 (brs, 1H), 5.05 - 4.7 (3H, m), 3.8 - 3.3 (2H, m), 2.9 (brs, 1H), 2.18 (3H, s), 2.12 (3H, s), 1.9 - 1.6 (4H, m), 1.39 (3H, s).

### Example 5: Preparation of (S)-(-)-6-hydroxy-2,5,7,8-tetramethylchroman-2-ethanol

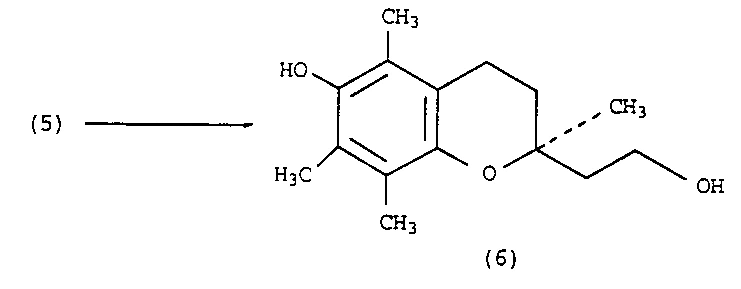

In an atmosphere of a hydrogen stream, the crude tricyclic ketal compound (5) prepared in Example 4 was dissolved in 10 ml of methyl alcohol, and 30 mg of palladium (II) hydroxide and 5 drops of trichloromethane were added. The reaction mixture was stirred at a room temperature for 3 days, filtered with celite. The solvent was evaporated under a reduced pressure, and the residue was treated by silica gel column chromatography. Then, 215 g (70 %) of the above-mentioned compound (6) was obtained from the ether/hexane (1:2) effluent as a brown solid, and after recrystallization from dichloromethane/hexane, a colorless crystal was obtained.
Mass analysis (m/e): 250 (M+), 164 (100 %).
IR (neat) cm⁻¹: 3400.
¹H-NMR (CDCl₃) δ: 4.7 (1H, brs), 3.90 (2H, t, J= 7Hz), 2.66 (2H, t, J=7Hz), 2.16 (3H, s), 2.10 (6H, s), 1.7 - 2.2 (4H, m), 1.28 (3H, s).
[α]_{D}²⁷ = -4.06 (c=0.7, methyl alcohol).
Melting point: 137 - 138 °C.

## Claims

1. A process for manufacturing an optically active (S)- or (R)-chroman-2-ethanol compound of the general formula (I): 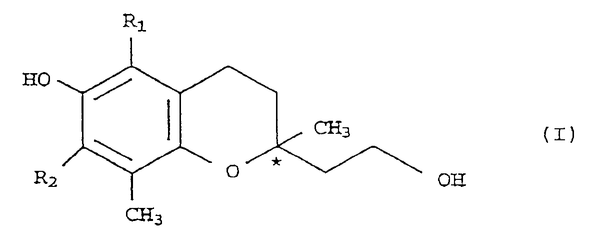 wherein R₁ and R₂ independently represent a hydrogen atom or a lower alkyl group having 1 to 4 carbon atoms, and the chiral central carbon atom marked with a symbol * in said formula (I) alternatively has one of an R-configuration and an S-configuration, said process comprising the following steps:
reducing an optically active (S)- or (R)-mevalonolactone of the formula (II): 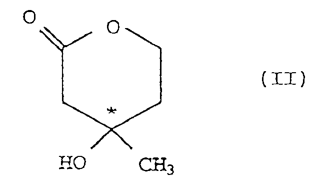 wherein the symbol * has the same meaning as above, to obtain the corresponding optically active (S)- or (R)-mevalonolactol of the formula (III): 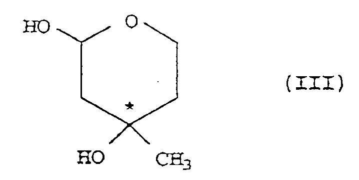 wherein the symbol * has the same meaning as above, while retaining the R-configuration or S-configuration of the chiral central carbon atom marked with the symbol * in said formula (II);
reacting the optically active (S)- or (R)-mevalonolactol of the formula (III) and a benzene magnesium halide of the general formula (IV): 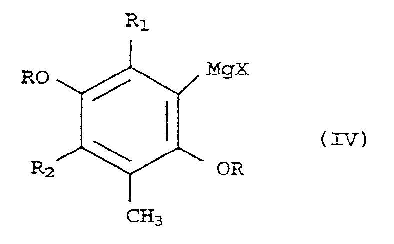 wherein R represents a lower alkyl group having 1 to 4 carbon atoms and optionally substituted with one or more lower alkoxy groups having 1 to 4 carbon atoms, a benzyl or substituted benzyl group, a substituted phenyl group, or a silyl group substituted with one or more lower alkyl groups having 1 to 4 carbon atoms, X represents a halogen atom, R₁ and R₂ have the same meanings as above, to obtain the corresponding optically active (S)- or (R)-phenylpentanetriol compound of the general formula (V): 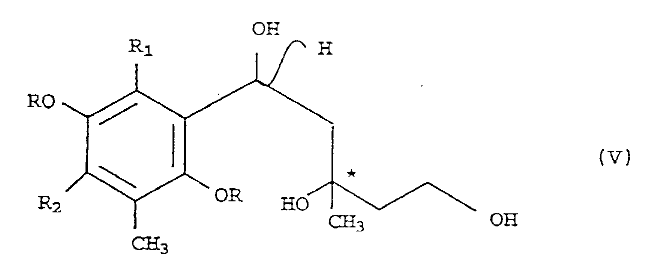 wherein R, R₁, R₂ and the symbol * have the same meanings as above, while retaining the R-configuration or S-configuration of the chiral central carbon atom marked with the symbol * in said formula (III);
removing the groups R from the optically active (S)- or (R)-phenylpentanetriol compound of the formula (V) to obtain the corresponding optically active (S)- or (R)-quinone-pentanetriol compound of the general formula (VI): 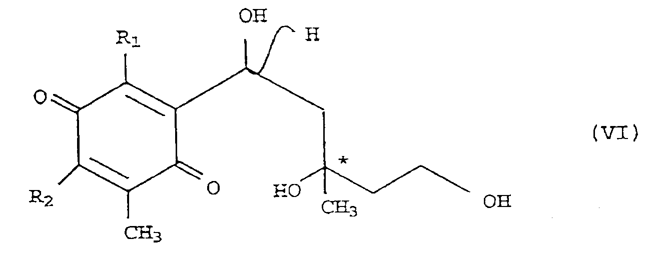 wherein R₁, R₂ and the symbol * have the same meanings as above, while retaining the R-configuration or S-configuration of the chiral central carbon atom marked with the symbol * in said formula (V);
cyclizing the optically active (S)- or (R)-quinonepentanetriol compound of the general formula (VI) to obtain the corresponding optically active tricyclic benzoquinone monoketal compound of the general formula (VII): 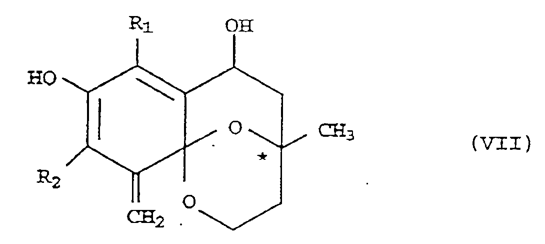 wherein R₁, R₂ and the symbol * have the same meanings as above, while retaining the R-configuration or S-configuration of the chiral central carbon atom marked with the symbol * in said formula (VI); and
treating the optically active tricyclic benzoquinone monoketal compound of a general formula (VII) under hydrogenolysis conditions, to obtain the corresponding optically active (S)- or (R)-chroman-2-ethanol compound, while retaining the R-configuration or S-configuration of the chiral central carbon atom marked with the symbol * in said formula (VII).

2. An optically active (S)- or (R)-phenylpentanetriol compound of the general formula (V) 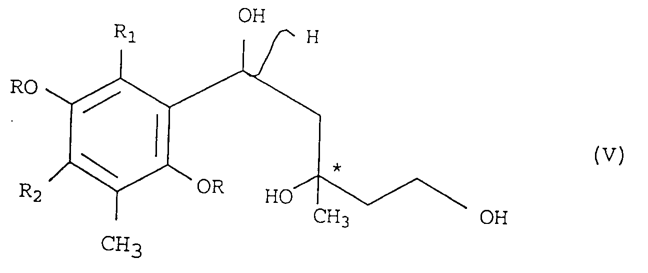 wherein R represents a lower alkyl group having 1 to 4 carbon atoms and optionally substituted with one or more lower alkoxy groups having 1 to 4 carbon atoms, a benzyl or substituted benzyl group, a substituted phenyl group or a silyl group substituted with one or more lower alkyl groups having 1 to 4 carbon atoms, R₁ and R₂ independently represent a hydrogen atom or a lower alkyl group having 1 to 4 carbon atoms, and the chiral central carbon atom marked with a symbol * in said formula (V) alternatively has one of an R-configuration and an S-configuration.

3. A process for manufacturing an optically active (S)- or (R)-phenylpentanetriol compound of the general formula (V) in the form of a mixture of two epimers 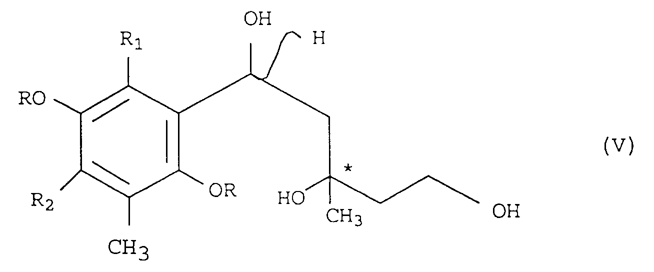 wherein R represents a lower alkyl group having 1 to 4 carbon atoms and optionally substituted with one or more lower alkoxy groups having 1 to 4 carbon atoms, a benzyl or substituted benzyl group, a substituted phenyl group or a silyl group substituted with one or more lower alkyl groups having 1 to 4 carbon atoms, R₁ and R₂ independently represent a hydrogen atom or a lower alkyl group having 1 to 4 carbon atoms, and the chiral central carbon atom marked with a symbol * in said formula (V) alternatively has one of an R-configuration and an S-configuration, said process comprising reacting an optically active (S)- or (R)-mevalonolactol compound of the formula (III) 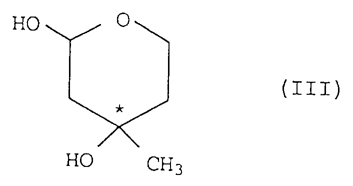 and a benzene magnesium halide of the general formula (IV) 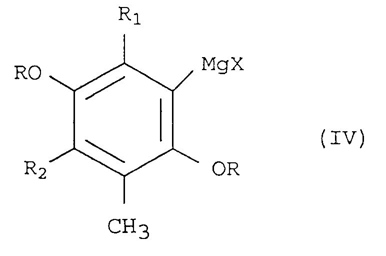 wherein R, R₁, R₂ and the symbol * have the same meanings as above and X represents a halogen atom, to obtain the corresponding optically active (S)- or (R)-phenylpentane-triol compound while retaining the R-configuration or S-configuration of the chiral central carbon atom marked with the symbol * in said formula (III).

4. An optically active (S)- or (R)-quinonepentanetriol compound of the general formula (VI) in the form of a mixture of two epimers 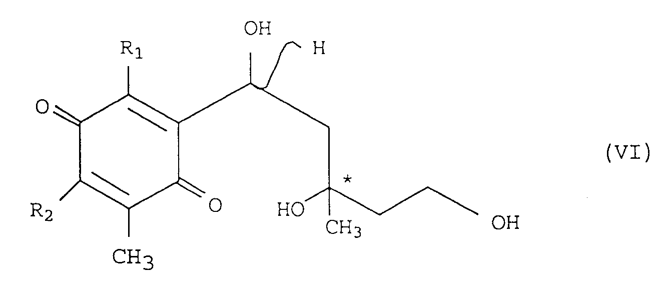 wherein R₁ and R₂ independently represent a hydrogen atom or a lower alkyl group having 1 to 4 carbon atoms, and the chiral central carbon atom marked with a symbol * in said formula (V) alternatively has one of an R-configuration and an S-configuration.

5. A process for manufacturing an optically active (S)- or (R)-quinonepentanetriol compound of the general formula (VI) 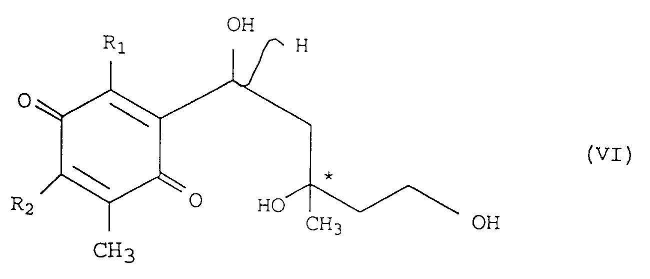 wherein R₁ and R₂ independently represent a hydrogen atom or a lower alkyl group having 1 to 4 carbon atoms, and the chiral central carbon atom marked with a symbol * in said formula (V) alternatively has one of an R-configuration and an S-configuration, said process comprising removing groups R for protecting hydroxy groups, from an optically active (S)- or (R)-phenylpentanetriol compound of the formula (V) 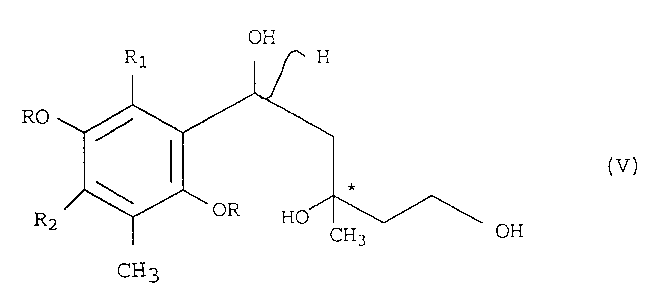 wherein R represents a lower alkyl group having 1 to 4 carbon atoms and optionally substituted with one or more lower alkoxy groups having 1 to 4 carbon atoms, a benzyl or substituted benzyl group, a substituted phenyl group or a silyl group substituted with one or more lower alkyl groups having 1 to 4 carbon atoms, R₁ and R₂ and the symbol * have the same meanings as above, to obtain the corresponding optically active (S)- or (R)-quinonepentanetriol compound while retaining the R-configuration or S-configuration of the chiral central carbon atom marked with the symbol * in said formula (V).

6. An optically active tricyclic benzoquinone monoketal compound of the general formula (VII) 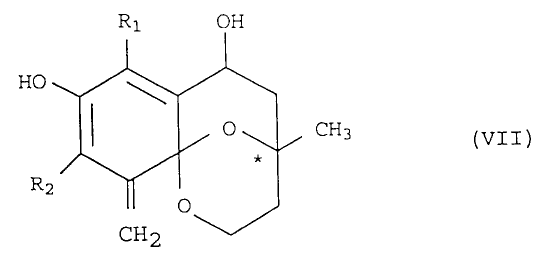 wherein R₁ and R₂ independently represent a hydrogen atom or a lower alkyl group having 1 to 4 carbon atoms, and the chiral central carbon atom marked with a symbol * in said formula (VII) alternatively has one of an R-configuration and an S-configuration.

7. A process for manufacturing an optically active tricyclic benzoquinone monoketal compound of the general formula (VII) 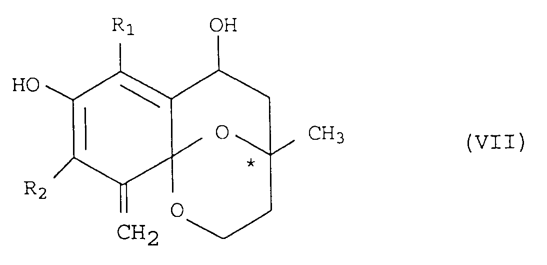 wherein R₁ and R₂ independently represent a hydrogen atom or a lower alkyl group having 1 to 4 carbon atoms, and the chiral central carbon atom marked with a symbol * in said formula (VII) alternatively has one of an R-configuration and an S-configuration, said process comprising cyclizing an optically active (S)- or (R)-quinonepentanetriol compound of the general formula (VI) 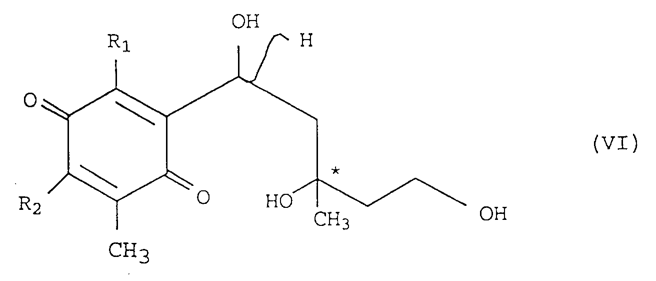 wherein R₁ and R₂ and the symbol * have the same meanings as above, to obtain the corresponding optically active tricyclic benzoquinone monoketal compound while retaining the R-configuration or S-configuration of the chiral central carbon atom marked with the symbol * in said formula (VI).

## Patentansprüche

1. Verfahren zur Herstellung einer optisch aktiven (S)-oder (R)-Chroman-2-ethanol-Verbindung der allgemeinen Formel (I) 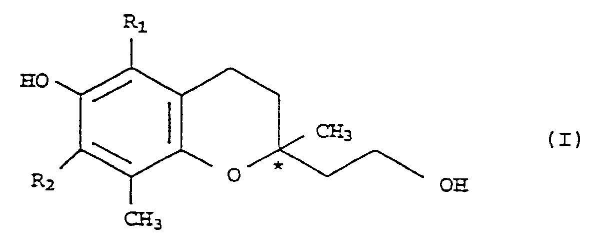 wobei R₁ und R₂, unabhängig voneinander, ein Wasserstoffatom oder einen niederen Alkylrest mit 1 bis 4 Kohlenstoffatomen bedeuten, und das in Formel (I) mit einem Symbol * markierte zentrale chirale Kohlenstoffatom entweder eine R-Konfiguration oder eine S-Konfiguration besitzt,
die folgenden Stufen umfassend:
Reduktion eines optisch aktiven (S)- oder (R)-Mevalonolactons der Formel (II): 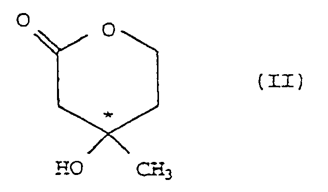 wobei das Symbol * die vorstehend angegebene Bedeutung besitzt, wobei das entsprechende optisch aktive (S)-oder (R)-Mevalonolactol der Formel (III) erhalten wird: 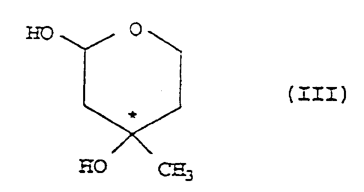 wobei das Symbol * die vorstehend angegebene Bedeutung besitzt, und dabei die R-Konfiguration oder S-Konfiguration des in der Formel (II) mit dem Symbol * markierten, zentralen chiralen Kohlenstoffatoms erhalten bleibt;
Umsetzung des optisch aktiven (S)- oder (R)-Mevalonolactols der Formel (III) und eines Benzolmagnesiumhalogenids der allgemeinen Formel (IV): 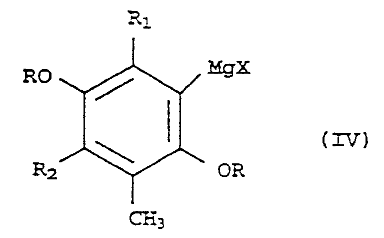 wobei R einen niederen Alkylrest mit 1 bis 4 Kohlenstoffatomen, der gegebenenfalls mit einem oder mehreren niederen Alkoxyresten mit 1 bis 4 Kohlenstoffatomen substituiert ist, einen Benzyl- oder substituierten Benzylrest, einen substituierten Phenylrest, oder einen mit einem oder mehreren niederen Alkylresten, die 1 bis 4 Kohlenstoffatome aufweisen, substituierten Silylrest, bedeutet, X ein Halogenatom bedeutet, R₁ und R₂ die vorstehend angegebenen Bedeutungen besitzen, wobei die entsprechende optisch aktive (S)- oder (R)-Phenylpentantriolverbindung der allgemeinen Formel (V) erhalten wird: 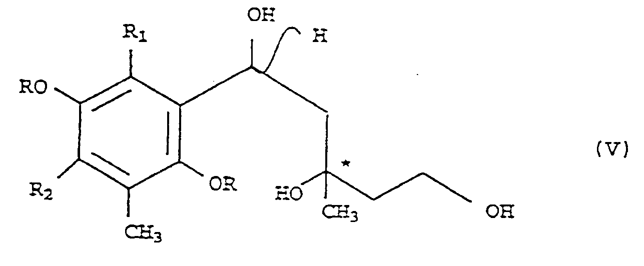 wobei R, R₁, R₂ und das Symbol * die vorstehend angegebenen Bedeutungen besitzen, und dabei die R-Konfiguration oder die S-Konfiguration des in der Formel (III) mit dem Symbol * markierten zentralen chiralen Kohlenstoffatoms erhalten bleibt;
Entfernen der Reste R aus der optisch aktiven (S)- oder (R)-Phenylpentantriolverbindung der Formel (V), wobei die entsprechende optisch aktive (S)- oder (R)-Chinonpentantriolverbindung der allgemeinen Formel (VI) erhalten wird: 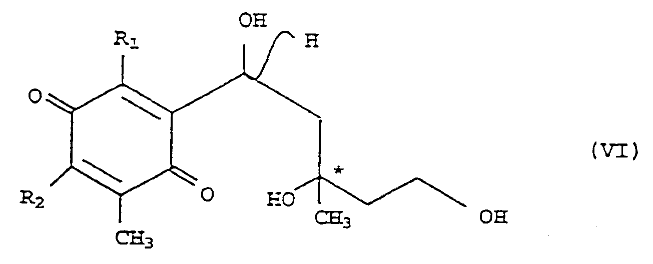 wobei R₁, R₂ und das Symbol * die vorstehend angegebenen Bedeutungen besitzen, und dabei die R-Konfiguration oder die S-Konfiguration des in Formel (V) mit dem Symbol * markierten zentralen chiralen Kohlenstoffatoms erhalten bleibt;
Cyclisieren der optisch aktiven (S)- oder (R)-Chinonpentantriolverbindung der allgemeinen Formel (VI), wobei die entsprechende optisch aktive tricyclische Benzochinonmonoketalverbindung der allgemeinen Formel (VII) erhalten wird: 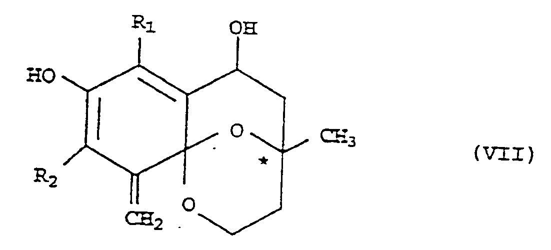 wobei R₁, R₂ und das Symbol * die vorstehend angegebenen Bedeutungen besitzen, und dabei die R-Konfiguration oder die S-Konfiguration des in der Formel (VI) mit dem Symbol * markierten zentralen chiralen Kohlenstoffatoms erhalten bleibt; und
Behandlung der optisch aktiven tricyclischen Benzochinonmonoketalverbindung der allgemeinen Formel (VII) unter Hydrogenolysebedingungen, wobei die entsprechende optisch aktive (S)- oder (R)-Chroman-2-ethanol-Verbindung erhalten wird, wobei die R-Konfiguration oder S-Konfiguration des in der Formel (VII) mit dem Symbol * markierten zentralen chiralen Kohlenstoffatoms erhalten bleibt.

2. Optisch aktive (S)- oder (R)-Phenylpentantriolverbindung der allgemeinen Formel (V) 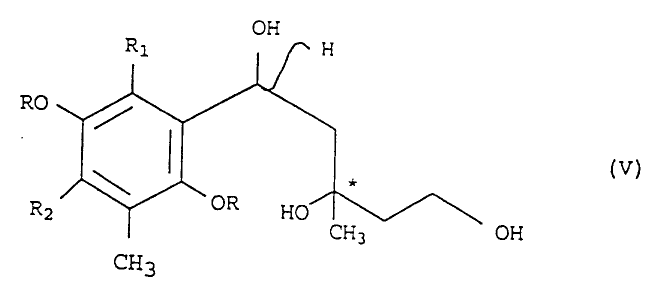 wobei R einen niederen Alkylrest mit 1 bis 4 Kohlenstoffatomen, der gegebenenfalls mit einem oder mehreren niederen Alkoxyresten mit 1 bis 4 Kohlenstoffatomen substituiert ist, einen Benzyl- oder substituierten Benzylrest, einen substituierten Phenylrest oder einen mit einem oder mehreren niederen Alkylresten, die 1 bis 4 Kohlenstoffatome aufweisen, substituierten Silylrest, bedeutet,
R₁ und R₂, unabhängig voneinander, ein Wasserstoffatom oder einen niederen Alkylrest mit 1 bis 4 Kohlenstoffatomen darstellen und das in der Formel (V) mit einem Symbol * markierte zentrale chirale Kohlenstoffatom entweder eine R-Konfiguration oder eine S-Konfiguration besitzt.

3. Verfahren zur Herstellung einer optisch aktiven (S)-oder (R)-Phenylpentantriolverbindung der allgemeinen Formel (V) in Form eines Gemisches von zwei Epimeren 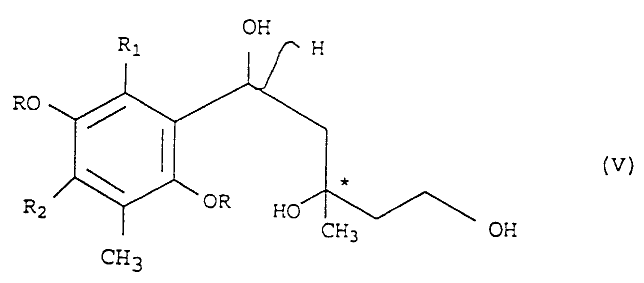 wobei R einen niederen Alkylrest mit 1 bis 4 Kohlenstoffatomen, der gegebenenfalls mit einem oder mehreren niederen Alkoxyresten mit 1 bis 4 Kohlenstoffatomen substituiert ist, einen Benzyl- oder substituierten Benzylrest, einen substituierten Phenylrest oder einen mit einem oder mehreren niederen Alkylresten, die 1 bis 4 Kohlenstoffatome aufweisen, substituierten Silylrest bedeutet, R₁ und R₂, unabhängig voneinander, ein Wasserstoffatom oder einen niederen Alkylrest mit 1 bis 4 Kohlenstoffatomen bedeuten, und das in Formel (V) mit einem Symbol * markierte zentrale chirale Kohlenstoffatom entweder eine R-Konfiguration oder eine S-Konfiguration besitzt,
umfassend die Umsetzung einer optisch aktiven (S)- oder (R)-Mevalonolactolverbindung der Formel (III) 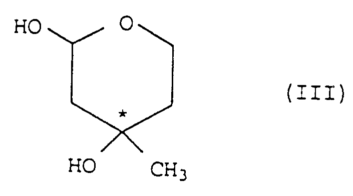 und eines Benzolmagnesiumhalogenids der allgemeinen Formel (IV) 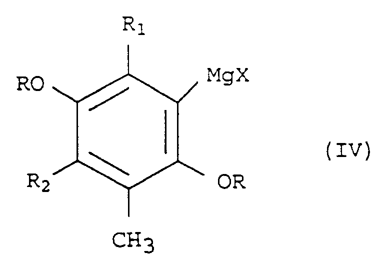 wobei R, R₁, R₂ und das Symbol * die vorstehend angegebenen Bedeutungen besitzen und X ein Halogenatom bedeutet, wobei die entsprechende optisch aktive (S)- oder (R)-Phenylpentantriolverbindung erhalten wird, und die R-Konfiguration oder S-Konfiguration des in Formel (III) mit dem Symbol * markierten zentralen chiralen Kohlenstoffatoms erhalten bleibt.

4. Optisch aktive (S)- oder (R)-Chinonpentantriolverbindung der allgemeinen Formel (VI) in Form eines Gemisches von zwei Epimeren 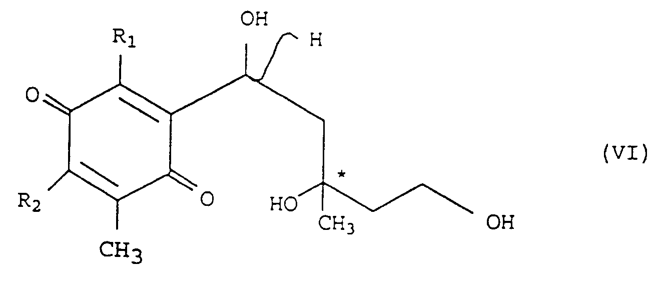 wobei R₁ und R₂, unabhängig voneinander, ein Wasserstoffatom oder einen niederen Alkylrest mit 1 bis 4 Kohlenstoffatomen bedeuten, und das in Formel (V) mit einem Symbol * markierte zentrale chirale Kohlenstoffatom entweder eine R-Konfiguration oder eine S-Konfiguration besitzt.

5. Verfahren zur Herstellung einer optisch aktiven (S)-oder (R)-Chinonpentantriolverbindung der allgemeinen Formel (VI) 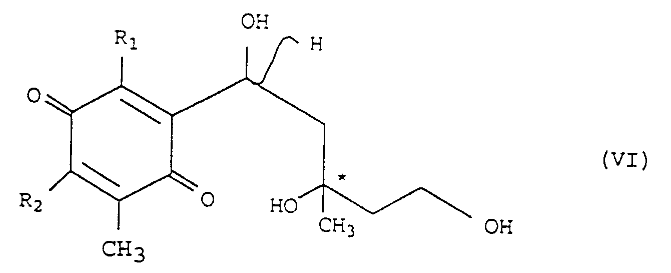 wobei R₁ und R₂, unabhängig voneinander, ein Wasserstoffatom oder einen niederen Alkylrest mit 1 bis 4 Kohlenstoffatomen bedeuten und das in Formel (V) mit einem Symbol * markierte zentrale chirale Kohlenstoffatom entweder eine R-Konfiguration oder S-Konfiguration besitzt,
umfassend das Entfernen der Reste R zum Schutze der Hydroxygruppen aus einer optisch aktiven (S)- oder (R)-Phenylpentantriolverbindung der Formel (V) 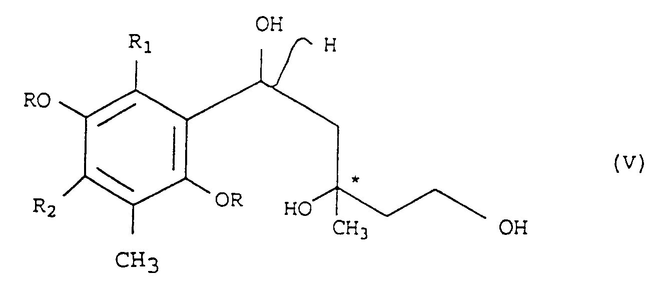 wobei R einen niederen Alkylrest mit 1 bis 4 Kohlenstoffatomen, der gegebenenfalls mit einem oder mehreren niederen Alkoxyresten mit 1 bis 4 Kohlenstoffatomen substituiert ist, einen Benzyl- oder substituierten Benzylrest, einen substituierten Phenylrest oder einen mit einem oder mehreren niederen Alkylresten, die 1 bis 4 Kohlenstoffatome aufweisen, substituierten Silylrest bedeutet, R₁ und R₂ und das Symbol * die vorstehend angegebenen Bedeutungen besitzen, wobei die entsprechende optisch aktive (S)- oder (R)-Chinonpentantriolverbindung erhalten wird, und dabei die R-Konfiguration oder S-Konfiguration des in der Formel (V) mit dem Symbol * markierte zentrale chirale Kohlenstoffatom erhalten bleibt.

6. Optisch aktive tricyclische Benzochinonmonoketalverbindung der allgemeinen Formel (VII) 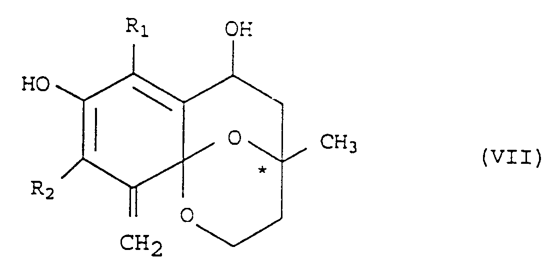 wobei R₁ und R₂ unabhängig voneinander ein Wasserstoffatom oder einen niederen Alkylrest mit 1 bis 4 Kohlenstoffatomen bedeuten, und das in der Formel (VII) mit dem Symbol * markierte zentrale chirale Kohlenstoffatom entweder eine R-Konfiguration oder eine S-Konfiguration besitzt.

7. Verfahren zur Herstellung einer optisch aktiven tricyclischen Benzochinonmonoketalverbindung der allgemeinen Formel (VII) 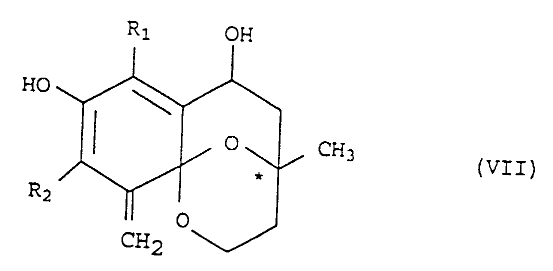 wobei R₁ und R₂, unabhängig voneinander, ein Wasserstoffatom oder einen niederen Alkylrest mit 1 bis 4 Kohlenstoffatomen bedeuten und das in der Formel (VII) mit einem Symbol * markierte zentrale chirale Kohlenstoffatom entweder eine R-Konfiguration oder eine S-Konfiguration besitzt,
umfassend das Cyclisieren einer optisch aktiven (S)-oder (R)-Chinonpentantriolverbindung der allgemeinen Formel (VI) 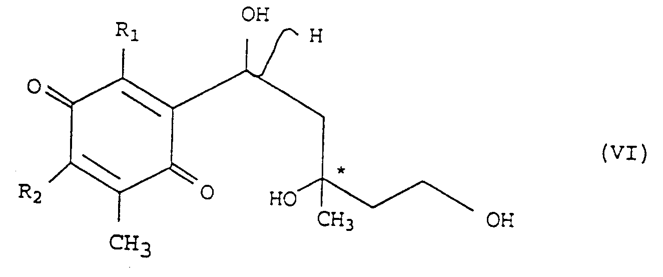 wobei R₁ und R₂ und das Symbol * die vorstehend angegebenen Bedeutungen besitzen, wobei die entsprechende optisch aktive tricyclische Benzochinonmonoketalverbindung erhalten wird, und dabei die R-Konfiguration oder S-Konfiguration des in Formel (VI) mit dem Symbol * markierten zentralen chiralen Kohlenstoffatoms erhalten bleibt.

## Revendications

1. Procédé pour la préparation d'un (S)- ou (R)-chromanne-2-éthanol optiquement actif de formule générale (I) : 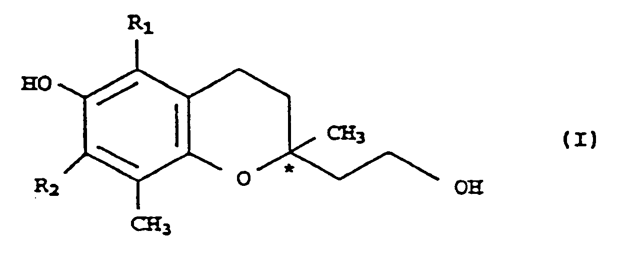 dans laquelle R₁ et R₂ indépendamment représentent un atome d'hydrogène ou un groupe alkyle inférieur ayant 1 à 4 atomes de carbone et l'atome de carbone central chiral marque d'un symbole * dans ladite formule (I) a la configuration R ou la configuration S, ledit procédé comprenant les étapes suivantes :
réduction d'une (S)- ou (R)-mévalonolactone optiquement active de formule (II) : 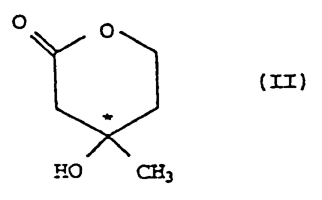 dans laquelle le symbole * a la même signification que ci-dessus, pour obtenir le (S)- ou (R)-mévalonolactol optiquement actif correspondant de formule (III) : 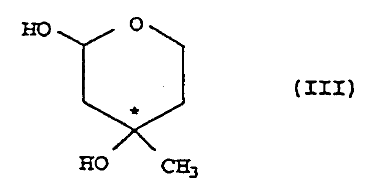 dans laquelle le symbole * a la même signification que ci-dessus, tout en conservant la configuration R ou la configuration S de l'atome de carbone central chiral marqué du symbole * dans ladite formule (II) ;
réaction du (S)- ou (R)-mévalonolactol optiquement actif de formule (III) et d'un halogénure de benzène-magnésium de formule générale (IV) : 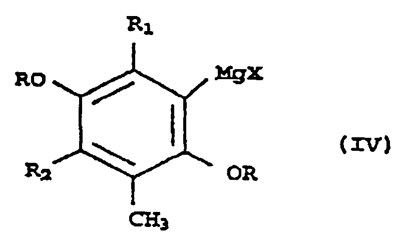 dans laquelle R représente un groupe alkyle inférieur ayant 1 à 4 atomes de carbone et facultativement substitué avec un ou plusieurs groupes alcoxy inférieurs ayant 1 à 4 atomes de carbone, un groupe benzyle ou un groupe benzyle substitué, un groupe phényle substitué ou un groupe silyle substitué avec un ou plusieurs groupes alkyles inférieurs ayant 1 à 4 atomes de carbone, X représente un atome d'halogène et R₁ et R₂ ont la même signification que ci-dessus, pour obtenir le (S)- ou (R)-phénylpentanetriol optiquement actif correspondant de formule générale (V) : 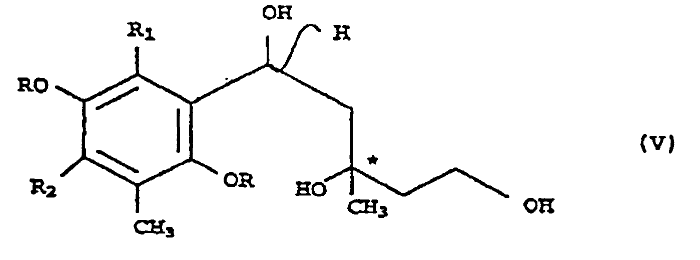 dans laquelle R, R₁, R₂ et le symbole * ont les mêmes significations que ci-dessus, tout en conservant la configuration R ou la configuration S de l'atome de carbone central chiral marqué du symbole * dans ladite formule (III) ;
élimination des groupes R du (S)- ou (R)-phénylpentanetriol optiquement actif de formule (V) pour obtenir le (S)- ou (R)-quinonepentanetriol optiquement actif correspondant de formule générale (VI) : 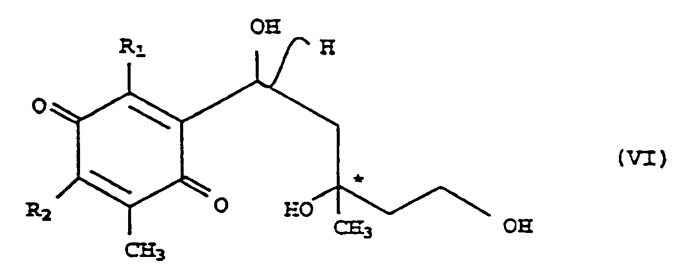 dans laquelle R₁, R₂ et le symbole * ont les mêmes significations que ci-dessus, tout en conservant la configuration R ou la configuration S de l'atome de carbone central chiral marqué du symbole * dans ladite formule (V) ;
cyclisation du (S)- ou (R)-quinonepentanetriol optiquement actif de formule générale (VI) pour obtenir le benzoquinone-monoacétal tricyclique optiquement actif correspondant de formule générale (VII) : 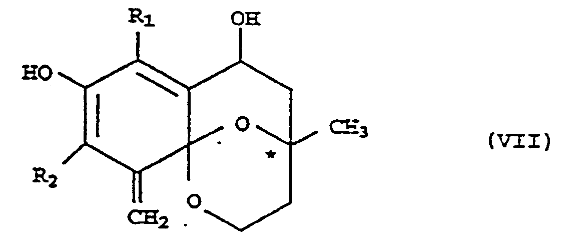 dans laquelle R₁, R₂ et le symbole * ont les mêmes significations que ci-dessus, tout en conservant la configuration R ou la configuration S de l'atome de carbone central chiral marqué du symbole * dans ladite formule (VI) ; et
traitement du benzoquinone-monoacétal tricyclique optiquement actif de formule générale (VII) dans des conditions d'hydrogénolyse pour obtenir le (S)- ou (R)-chromanne-2-éthanol optiquement actif correspondant, tout en conservant la configuration R ou la configuration S de l'atome de carbone central chiral marqué du symbole * dans ladite formule (VII).

2. (S)- ou (R)-phénylpentanetriol optiquement actif de formule générale (V) : 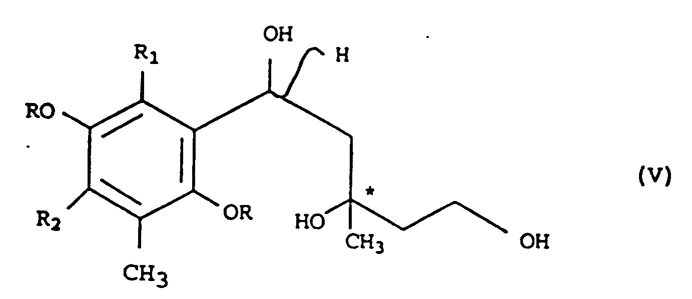 dans laquelle R représente un groupe alkyle inférieur ayant de 1 à 4 atomes de carbone et facultativement substitué avec un ou plusieurs groupes alcoxy inférieurs ayant 1 à 4 atomes de carbone, un groupe benzyle ou un groupe benzyle substitué, un groupe phényle substitué ou un groupe silyle substitué avec un ou plusieurs groupes alkyles inférieurs ayant 1 à 4 atomes de carbone, R₁ et R₂ représentent indépendamment un atome d'hydrogène ou un groupe alkyle inférieur ayant 1 à 4 atomes de carbone et l'atome de carbone central chiral marqué d'un symbole * dans ladite formule (V) a l'une ou l'autre de la configuration R et de la configuration S.

3. Procédé pour la préparation d'un (S)- ou (R)-phényl-pentanetriol optiquement actif de formule générale (V) sous forme d'un mélange de deux épimères : 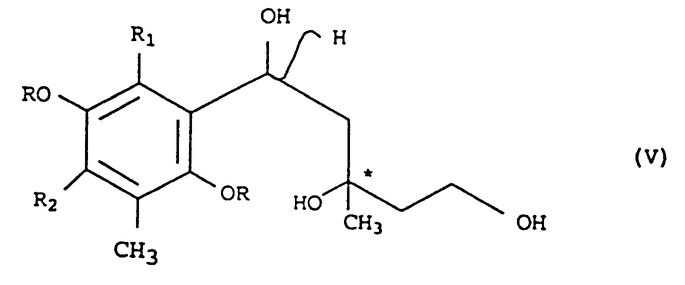 dans laquelle R représente un groupe alkyle inférieur ayant 1 à 4 atomes de carbone et facultativement substitué avec un ou plusieurs groupes alcoxy inférieurs ayant 1 à 4 atomes de carbone, un groupe benzyle ou un groupe benzyle substitué, un groupe phényle substitué ou un groupe silyle substitué avec un ou plusieurs groupes alkyles inférieurs ayant 1 à 4 atomes de carbone, R₁ et R₂ représentent indépendamment un atome d'hydrogène ou un groupe alkyle inférieur ayant 1 à 4 atomes de carbone et l'atome de carbone central chiral marqué d'un symbole * dans ladite formule (V) a l'une ou l'autre de la configuration R et de la configuration S, ledit procédé comprenant la réaction d'un (S)- ou (R)-mévalonolactol optiquement actif de formule (III) : 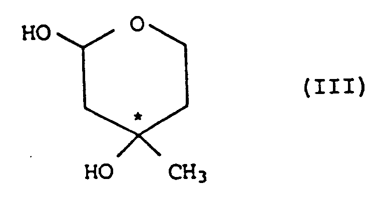 et d'un halogénure de benzène-magnésium de formule générale (IV) : 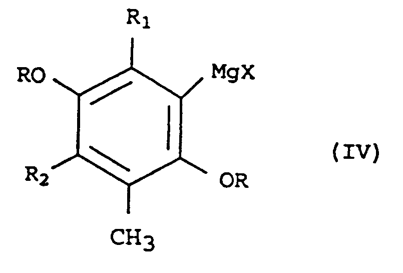 dans laquelle R₁, R₂ et le symbole * ont les mêmes significations que ci-dessus et X représente un atome d'halogène, pour obtenir le (S)-ou (R)-phénylpentanetriol optiquement actif correspondant, en conservant la configuration R ou la configuration S de l'atome de carbone central chiral marqué du symbole * dans ladite formule (III).

4. (S)- ou (R)-quinonepentanetriol optiquement actif de formule générale (VI) sous forme d'un mélange de deux épimères : 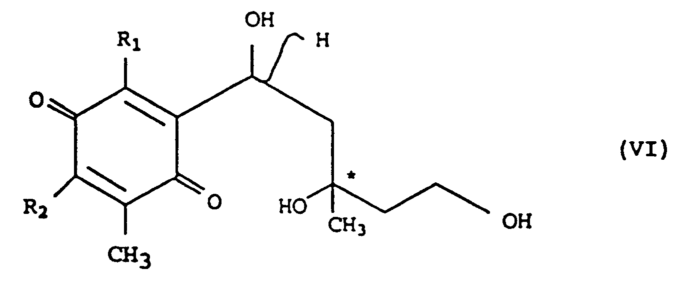 où R₁ et R₂ indépendamment représentent un atome d'hydrogène ou un groupe alkyle inférieur ayant 1 à 4 atomes de carbone et l'atome de carbone central chiral marqué d'un symbole * dans ladite formule (V) a l'une ou l'autre de la configuration R et de la configuration S.

5. Procédé pour la préparation d'un (S)- ou (R)-quinonepentanetriol de formule générale (VI) : 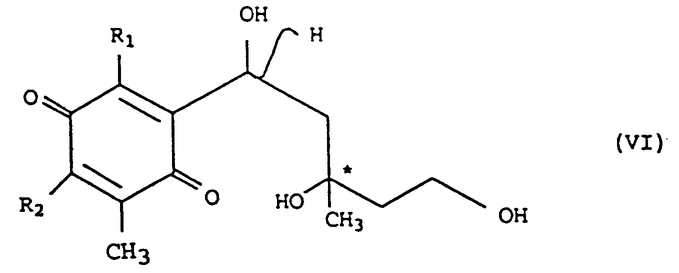 dans laquelle R₁, R₂ indépendamment représentent un atome d' hydrogène ou un groupe alkyle inférieur ayant 1 à 4 atomes de carbone et l'atome de carbone central chiral marqué d'un symbole * dans ladite formule (V) a l'une ou l'autre de la configuration R et de la configuration S, ledit procédé comprenant l'élimination des groupes R protégeant les groupes hydroxy d'un (S)- ou (R)-phénylpentanetriol optiquement actif de formule (V) : 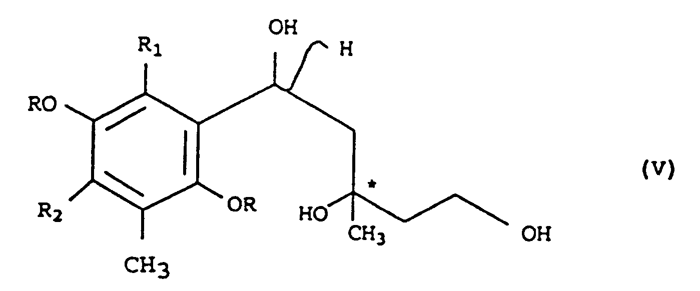 dans laquelle R représente un groupe alkyle inférieur ayant 1 à 4 atomes de carbone et facultativement substitué avec un ou plusieurs groupes alcoxy inférieurs ayant 1 à 4 atomes de carbone, un groupe benzyle ou un groupe benzyle substitué, un groupe phényle substitué ou un groupe silyle substitué avec un ou plusieurs groupes alkyles inférieurs ayant 1 à 4 atomes de carbone, R₁ et R₂ et le symbole * ont les mêmes significations que ci-dessus, pour obtenir le (S)- ou (R)-quinonepentanetriol optiquement actif correspondant, en conservant la configuration R ou la configuration S de l'atome de carbone central chiral marqué du symbole * dans ladite formule (V).

6. Benzoquinone-monoacétal tricyclique optiquement actif de formule générale (VII) : 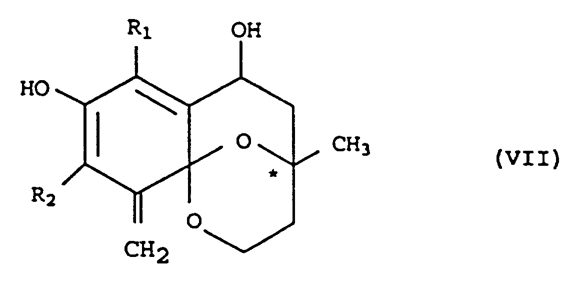 dans laquelle R₁ et R₂ indépendamment représentent un atome d'hydrogène ou un groupe alkyle inférieur ayant 1 à 4 atomes de carbone et l'atome de carbone central chiral marqué d'un symbole * dans ladite formule (VII) a l'une ou l'autre de la configuration R et de la configuration S.

7. Procédé pour la préparation d'un benzoquinone-monoacétal tricyclique optiquement actif de formule générale (VII) : 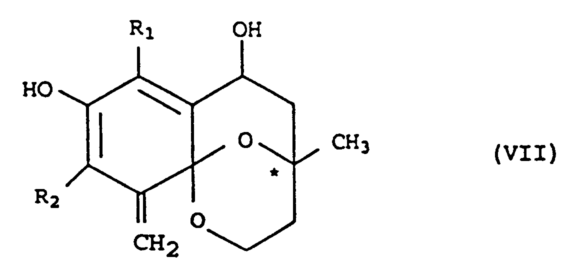 dans laquelle R₁ et R₂ indépendamment représentent un atome d'hydrogène ou un groupe alkyle inférieur ayant 1 à 4 atomes de carbone et l'atome de carbone central chiral marqué d'un symbole * dans ladite formule (VII) a l'une ou l'autre de la configuration R et de la configuration S, ledit procédé comprenant la cyclisation d'un (S)- ou (R)-quinonepentanetriol optiquement actif de formule générale (VI) : 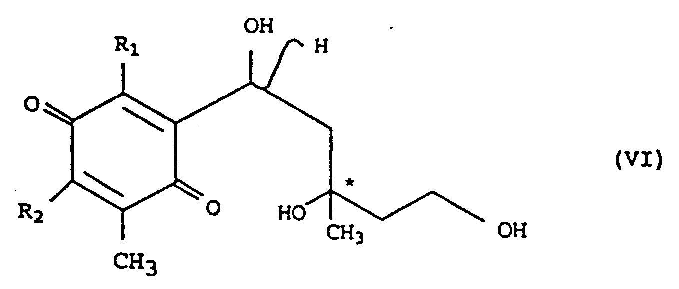 dans laquelle R₁ et R₂ et le symbole * ont les mêmes significations que ci-dessus, pour obtenir le benzoquinone-monoacétal tricyclique optiquement actif correspondant, tout en conservant la configuration R ou la configuration S de l'atome de carbone central chiral marqué du symbole * dans ladite formule (VI).
